# EUROPEAN PATENT SPECIFICATION

(11) **EP 1 328 814 B1**
(45) Date of publication and mention of the grant of the patent: **11.10.2006**
(21) Application number: 01983776.4
(22) Date of filing: 22.10.2001
(51) Int. Cl.: G01N 33/68, C12N 15/10

(54) **METHOD FOR IN VIVO IDENTIFICATION OF INTRACELLULAR EPITOPES**
METHODE ZUR IN VIVO IDENTIFIZIERUNG VON INTRAZELLULAREN EPITOPEN
PROCEDE D'IDENTIFICATION IN VIVO D'EPITOPES INTRACELLULAIRES

(30) Priority: 24.10.2000 IT RM20000561
(43) Date of publication of application: 23.07.2003
(73) Proprietor: S.I.S.S.A. Scuola Internazionale Superiore di Studi Avanzati, 34014 Trieste (IT)
(72) Inventor: CATTANEO, Antonino, S.I.S.S.A. Scuola Intern., I-34014 Trieste (IT); VISINTIN, Michela, S.I.S.S.A. Scuola Intern., I-34014 Trieste (IT)
(74) Representative: Capasso, Olga
(86) International application number: PCT/IT2001/000535
(87) International publication number: WO 2002/035237

(56) References cited:
- WO-A-01/48017
- GB-A- 2 344 886
- VISINTIN MICHELA ET AL: "Selection of antibodies for intracellular function using a two-hybrid in vivo system" PROCEEDINGS OF THE NATIONAL ACADEMY OF SCIENCES OF THE UNITED STATES, NATIONAL ACADEMY OF SCIENCE, WASHINGTON, DC, US, vol. 96, no. 21, 12 October 1999 (1999-10-12), pages 11723-11728, XP002143442 ISSN: 0027-8424 cited in the application
- PORTNER-TALIANA A ET AL: "In vivo selection of single-chain antibodies using a yeast two-hybrid system" JOURNAL OF IMMUNOLOGICAL METHODS, ELSEVIER SCIENCE PUBLISHERS B.V.,AMSTERDAM, NL, vol. 238, no. 1-2, April 2000 (2000-04), pages 161-172, XP004195472 ISSN: 0022-1759
- DE JAEGER GEERT ET AL: "Analysis of the interaction between single-chain variable fragments and their antigen in a reducing intracellular environment using the two-hybrid system." FEBS LETTERS., vol. 467, no. 2-3, 11 February 2000 (2000-02-11), pages 316-320, XP002205954 ISSN: 0014-5793
- FIELDS S ET AL: "THE TWO-HYBRID SYSTEM: AN ASSAY FOR PROTEIN-PROTEIN INTERACTIONS" TRENDS IN GENETICS, ELSEVIER SCIENCE PUBLISHERS B.V. AMSTERDAM, NL, vol. 10, no. 8, 1 August 1994 (1994-08-01), pages 286-292, XP000647708 ISSN: 0168-9525
- DATABASE BIOSIS [Online] BIOSCIENCES INFORMATION SERVICE, PHILADELPHIA, PA, US; 29 December 2000 (2000-12-29) CHEN ZHUI ET AL: "The C-terminal polylysine region and methylation of K-Ras are critical for the interaction between K-Ras and microtubules." Database accession no. PREV200100092316 XP002205955 & JOURNAL OF BIOLOGICAL CHEMISTRY, vol. 275, no. 52, 29 December 2000 (2000-12-29), pages 41251-41257, ISSN: 0021-9258

## Description

The present invention relates to a method for the *in vivo* identification of intracellular epitopes. More particularly the invention relates to a method for *in vivo* mapping epitopes of an antigen, which is normally present in a cell, using intracellular antibodies.

An essential element for efficiently utilize antibodies, as well as intracellular antibodies, is their epitope(s) identification, i.e. the antigen portion recognised by the antibody itself. In fact for research, diagnostic and therapy it is desirable to know the epitope of an antigen and select antibodies able to bind to the same.

Many different approaches have been used to map the epitopes of an antigen, all using *in vitro* technologies and very laborious procedures. The limitation of *in vitro* technologies derives from the fact that the antigen-antibody binding is not investigated in the natural functional context of the antigen, i.e. the intracellular environment.

A method (peptide scanning) makes use of: a) synthesis of peptides which overlap by few amino acids and, as a whole, comprise the complete amino acid sequence of the investigated antigen, and b) the challenging of each peptide with antibodies. The method is very time-consuming, laborious, expensive and, overall, not always provides satisfactory results. In fact not always an epitope recognised by an antibody consists of a linear amino acid sequence.

Presently the most useful technology for the identification of epitopes recognised by different antibodies is the use of peptides having random sequences, either selected from peptide-phage libraries or synthetically synthesized. The strategy further allows to identify peptides active as epitopes having no homology with the antigen primary sequence, thus resulting in a discontinuous or conformational epitope. The methodology, therefore, allows to map a virtual collection of epitopes recognised by a given antibody, even with no structural correlation with the starting antigen.

Alternatively mutated, truncated or deleted forms of the target antigen can be engineered, expressed in suitable cellular systems, purified and individually assayed. Obviously this procedure is very laborious too.

The authors of the present invention now provide a method which identifies *in vivo,* in the natural environment, epitopes of intracellular antigens by means of antibodies which are expressed and active in the cellular environment (intracellular antibodies).

The use of intracellular antibodies, i.e. antibodies or portions thereof, expressed and active within a cell, represents a successful technology to interact with the function of a protein, within its physiological intracellular environment (Cattaneo & Neuberger, 1987; Carlson, 1988; Biocca & Cattaneo, 1995; Cattaneo & Biocca, 1997; Cattaneo & Biocca, 1999; Marasco, 1997). Thus the technology allows to achieve the functional knock-out of the target protein. It is therefore possible to confer a given phenotype to a cell or organism by means of suitable intracellular antibodies.

The technology of intracellular antibodies is based on two advantageous aspects: 1) the virtually unlimited availability of the immune system (natural or artificial) repertoire, providing a source of molecules suitable for high affinity and specificity reactions against any protein, and 2) the possibility to direct a protein (and then an antibody) into different intracellular compartments by appropriated, autonomous and dominant intracellular localising signals.

The technology is validly used for research and biotechnology applications, particularly for gene therapy of human and animal pathologies, to provide experimental models of pathologies, and in plant biotechnology to produce pathogen resistant transgenic plants.

The recent big development of both human and other species genome sequencing technologies determined an increasing demand to provide technologies suitable to clarify the function of identified genes, thus resulting in a new research field, so called functional genomics. It includes methods and technologies suitable to identify gene functions in a way as possible as parallel, at high throughput and suitable to be automated. Presently this step represents a remarkable bottleneck for the industry and particularly for the procedures leading from genes to new classes of drugs.

Recently a development of the intracellular antibody technology was introduced, allowing its use as an elective methodology for programs of functional genomics: the so-called ITT (intrabody trap technology). The methodology allows to isolate or select, among all the antibodies directed against a target protein, the sub-population which is able to operate efficiently *in vivo,* in the intracellular environment. The technology is described in the International Patent Application PCT n. WO00/54057.

In summary it consists of a method to select antibodies from phage-libraries or from immune splenocytes, given their ability to bind an antigen in the intracellular environment. The method allows to overcome a limitation of the intracellular antibody technology, i.e. the fact that not all of antibodies able to bind *in vitro* an antigen are then able to bind the same antigen when expressed as cytoplasmic antibodies. Antibodies selected by the ITT procedure, based on the two-hybrid system, are able to fold correctly in the intracellular environment and to bind the target antigen therein. Therefore they are "validated" to be used as intracellular antibodies (Visintin et al., 1999). In all of ITT's applications, the antigen is challenged with an antibody library.

The authors of the present invention advantageously adapted and modified this technology for the identification of epitopes, i.e. the antigen portions able to recognise and bind an antibody. Such epitopes, in virtue of the selection technique, are *in vivo* recognised by the antibody itself. According to the technology of the invention, opposite to ITT, the antibody is the constant element to be challenged with a library, or a series of antigens. This new technique of *in vivo* epitope mapping of a specific intracellular antibody (IVEM) represents a valid alternative to the known *in vitro* techniques.

The simplicity of the technique makes the same applicable for large scale screenings. The advantage of the method results also from the fact that the epitope identification occurs *in vivo,.* without the need of peptide synthesis, but with the same if not higher accuracy than known methods. The method is able to accelerate the identification of epitopes to be useful for clinical, diagnostic and pharmacological applications, with large scale screeneings within a physiological contest.

Furthermore because it is becoming evident that active sites of a protein are more conserved than the whole structure, by means of the method of the invention, it is possible to identify ancestral proteins having similar functions.

Therefore, the method allows the *in vivo* identification of the epitopes of an intracellular protein. It is known that many intracellular proteins include structurally homologous domains or modules, involved in protein-protein (e.g. SH2, SH3, PH, WW, PTB, PDZ domains; Pawson, 1965) or protein-DNA interactions (e.g. zinc finger, homeo-box, helix-loop-helix, chromo domains, bromo-domains). Being able to know the antibody specificity for a domain with respect to similar domains belonging to other proteins represents an essential prerequisite of the applications designed for intracellular antibodies.

The information is essential also in all of more conventional uses of antibodies and the simplicity of the instant method provides an easy solution to this need. Accordingly an antibody of interest, targeting one of these protein modules or domains, is challenged with a library of protein domains of the same family.

Analogously the method of the invention allows to identify the specificity of a given antibody for a family of correlated proteins, as well as isoforms of a protein (i.e., isoforms resulting from alternative splicing), and the specificity of a given antibody for families of evolutionary correlated proteins. This allows phylogenetic studies to be carried out very easily.

A further very interesting application made feasible by the present invention is represented by the more easier isolation of specific antibodies for mutated forms of a given protein. In this aspect, the antigen library consists of a collection of point mutants of the antigenic protein of interest. Isolation of antibodies for mutated forms of tau intracellular proteins, present in neurodegenerative pathologies, as taupathies, (Hong, M., et al., 1998; Spillantini MG, et al., 1998; Goedert et al., 1999), or of p21-ras, present in many human cancers (Barbacid, 1987; Grand and Owen, 1991) can be carried out more easily than previously.

As exemplary experimental system the authors used tau protein, a protein involved in Alzheimer's disease. However the invention is directed to a general method for epitope identification.

It is therefore an object of the present invention a method for identification of epitopes of the tau protein able to bind intracellularly to a specific antibody comprising the steps of :
a) co-transforming cells by means of a first vector including the nucleotide sequence encoding for the region of an anti-tau antibody able to recognise and bind intracellularly the tau protein and by a second vector family, each member of the family comprising a nucleotide sequence encoding for a peptide and forming a peptide library as a whole;
b) growing co-transformed cells in such an environment that only cells wherein the antibody region and the peptide recognise and interact each other are able to replicate and/or be recognised because: the antibody region able to recognise and bind intracellularly the peptide is associated with a first molecule; the peptide is associated with a second molecule; the interaction between the first and the second molecule generates a selectable phenotype and/or a recognisable signal; and the interaction between the first and the second molecule occurs only when the antibody region and the peptide recognise and interact each other;
c) selecting cells and identifying the peptide as epitope.

According to a preferred embodiment the peptide library is a library of tau peptide fragments; or phylogenetic variants or pathology associated mutants or splicing isoforms thereof.

According to a preferred embodiment the cells are yeast cells.

The invention is now described according to preferred embodiments, with reference to the following figures:
figure 1 is a scheme of tau protein deletion mutants;
figure 2 shows western blot analysis of lexA-tau deletion mutant fusion proteins revealed by using a polyclonal anti-lexA antibody (panel a); a generic anti-tau monoclonal antibody (Tau-1) (panel a); another generic anti-tau monoclonal antibody (7.51) (panel c).
figure 3 shows the map of tau deletion mutants as described in Fasulo et al. 200 (panel a) and the anti-tau specificity of scFv2, scFv14 and scFv52 fragments, with respect to various deletion mutants *in vivo* (panel b). The epitope-scFv binding is detected by ITT technology measuring the transactivation of HIS3 and lacZ genes and therefore the ability of the cells to grow in medium without histidine and synthesise beta-galactosidase.

### Example 1 Tau mutants and fusion constructs with lexA

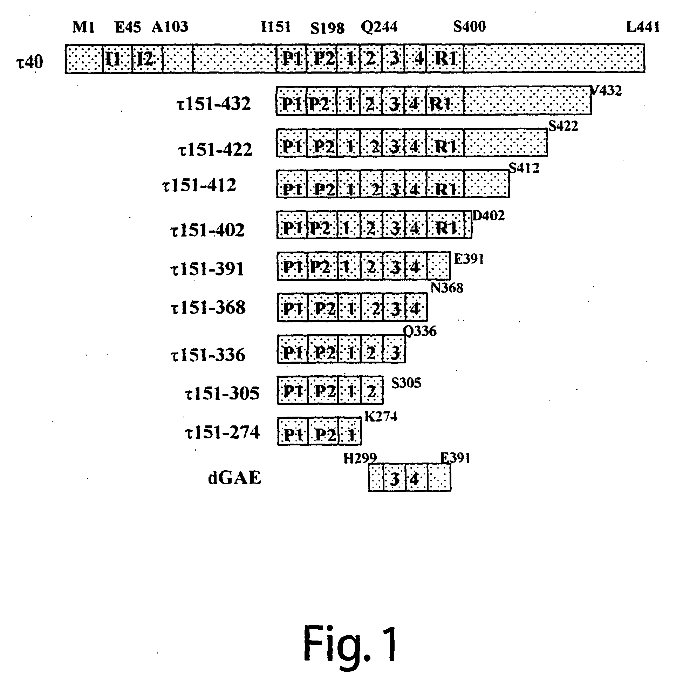

Deletion mutants of the tau protein were fused in frame to lexA binding domain. Such DNA binding domain is derived by an *E*. *coli* repressor protein (Brent & Ptashne, 1985) (Golemis & Brent, 1992) (Silver et al., 1986). LexA binding sites are located upstream to reporter genes, at the transcription activating region or promoter. The binding of lexA-tau deletion mutant proteins can reach and bind lexA binding sites but are unable to activate the transcription of reporter genes because of lacking of transcriptional activation domain. Tau fragments are illustrated in figure 1 and are:
- Tau 151-274 including the proline rich domain and the R1 repeated sequence able to bind to microtubules;
- Tau 151-305 including the proline rich domain and R1 and R2 repeated sequences able to bind to microtubules;
- Tau 151-336 including the proline rich domain and R1, R2 and R3 repeated sequences able to bind to microtubules;
- Tau 151-368 including the proline rich domain and R1, R2, R3 and R4 repeated sequences able to bind to microtubules;
- Tau 151-391 including the proline rich domain and R1, R2, R3 and R4 repeated sequences extended up to Glu 391 residue;
- Tau 151-402 including the proline rich domain and all the repeated sequences extended up to Asp 402 residue;
- Tau 151-412 including the proline rich domain and all the repeated sequences extended up to Ser 412 residue;
- Tau 151-422 including the proline rich domain and all the repeated sequences extended up to Ser 422 residue;
- dGAE including the minimal region constituting PHF filament core observed in Alzheimer's patients.

### Example 2 Transformation with Tau mutants and expression thereof

Plasmid DNA enconding lexA domain fused to tau protein deletion mutants is utilized to transform the L40 yeast strain (Hollenberg et al, 1995) using a standard method including lithium acetate to make permeable yeast cells. The method was provided by Clontech on the base of previously published protocols (Gietz et al., 1992; Hill et al., 1991; Schiestl & Gietz, 1989). Yeast cells are transformed using 0.1 µg of plasmid DNA and 100 µg of salmon sperm as carrier, in a solution including lithium acetate, polyethylene glycol and dimethyl sulphoxide. Transformed cells are plated on minimal medium (synthetic dropout) containing all the amino acids but tryptophan to identify the obtained phenotype. In fact the vector used to express lexA domain fused to tau deletion mutant proteins is able to complement the nutritional deficit resulting from the lack of tryptophan, thus allowing yeast growth without said amino acid.

Hybrid proteins produced by yeast cells are extracted from transformed yeast culture using an extraction buffer containing 10% β-mercaptethanol, 2% SDS, 0,1 % bromophenol blue and 10% glycerol (Sambrook et al., 1990).

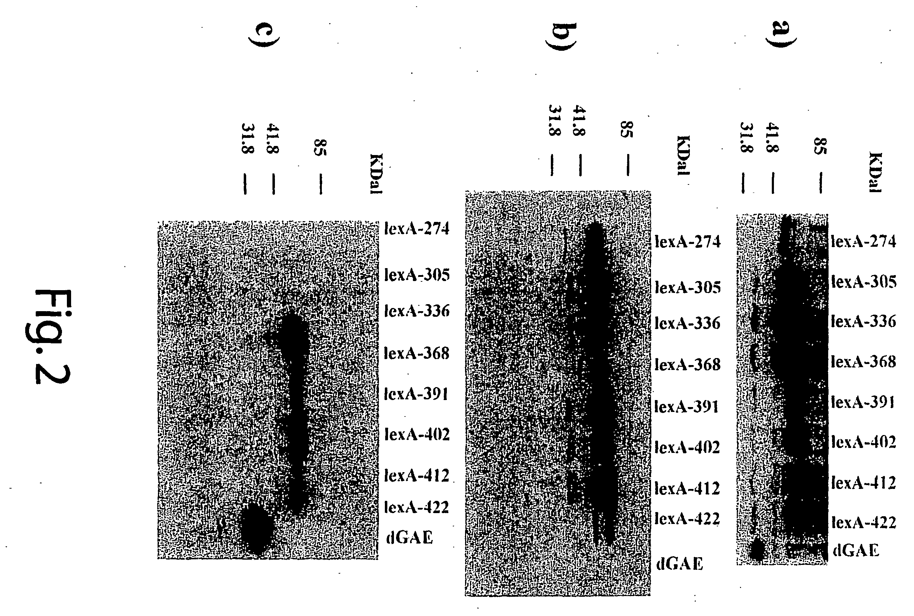

The expression level of fusion proteins was evaluated by western blot analysis, using anti lexA-protein polyclonal antibodies (Fig. 2a), anti Tau mAbs: Tau-1 (Roche) (Fig. 2b) and 7.51 (Novak et al., 1991) (Fig. 2c).

The experiment shows that tau deletion mutants are differently recognised by the two anti-tau antibodies, thus indicating that well expressed proteins have a suitable molecular weight and are then correctly translated and correctly express known epitopes, recognised by various used antibodies.
Example 3 Co-transformation of tau mutants with single chain (ScFv) anti-tau antibody fragments, selected by means of the ITT method
The experiment uses a panel of single chain Fv fragments (scFv) selected by ITT technology (Visintin et al., 1999 and PCT WO00/54057). A scFv fragment library exposed on the surface of filamentous bacteriophages was screened with the purified recombinant tau protein, bound to a solid phase. After two adsorption cycles, elution, growth of eluted phages in *E*. *coli,* the "polyclonal" population of anti-tau protein enriched antibody fragments is subcloned in the two-hybrid expression vector and tested for the tau protein binding ability in two-hybrid system. From yeast cells selected in the absence of histidine, anti-tau scFv antibody fragments were isolated, able to bind tau intracellularly.

Yeast colonies, transformed with tau mutants and with each of the ITT selected scFvs, were plated on medium selective for the identification of phenotype from nutritional reporter HIS3, which is under the control of lexA binding sites, whose expression allows cells to grow in the absence of histidine.

The reconstitution of the transcriptional factor by antigen-antibody specific interaction allows the transcription of both HIS3 and lacZ reporter genes. The identification of such interaction is performed by qualitative analysis of colonies grown in the absence of histidine, and by colour change (from white to blue) of the same colony in a colorimetric assay for β-galactosidase expression.

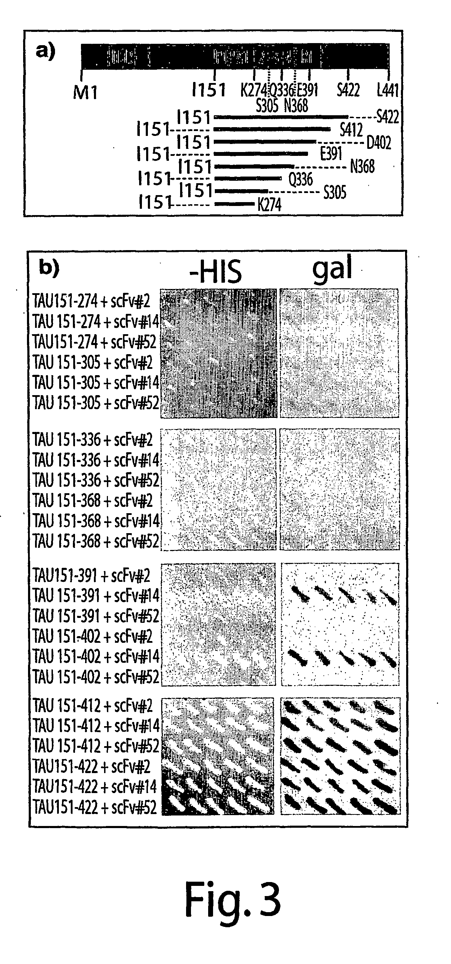

The specificity of three different scFv (2, 14, 52) fragments against the panel of tau antibody fragments is showed in Fig. 3 and summarised in Table 1.

**Table 1**

| Antigen | | | | | | | | |
|---|---|---|---|---|---|---|---|---|
| | | 151-274 | 151-305 | 151-336 | 151-368 | 151-391 | 151-402 | 151-412 |
| 151-422 dGAE | | | | | | | | |
| ScFv2 | | - | - | - | - | - | - | + |
| + | - | | | | | | | |
| ScFv14 | | - | - | - | - | + | + | + |
| + | + | | | | | | | |
| ScFv52 | | - | - | - | - | - | - | + |
| + | - | | | | | | | |

Fig. 3 shows the identification of tau protein epitopes by anti-tau single chain variable fragments, scFv2, scFv14 and scFv52. The growth on His⁻ dishes and the β-gal phenotype shows Tau peptides interacting with one or more of the three scFV fragments. In particular S412-S422 and N368-E391 fragments do contain an *in vivo* recognisable epitope.

### Example 4 Selection with the IACT method

According to the method as disclosed in the PCT application WO 00/54057, a series of 18 new anti tau ScFvs (A to Y) is selected and characterized by means of the same panel of Tau deletion mutants of the Example 1, but dGAE.

Each antibody has been challenged *in vivo* with each of Tau deletion mutants, according to the method of WO 00/54057. Results are shown in Table 2 and demonstrate that the method of the invention (IVEM) allowed the identification of three main regions (I 151-K 274; N 368-E 391; D 402-S 412) of Tau, recognized by antibodies selected with the IACT method.

**Table 2**

| **ScFv** | **TAU fragments** | | | | | | | |
|---|---|---|---|---|---|---|---|---|
| | **I 151-K 274** | **I 151-S 305** | **I 151-Q 336 N 368** | **I 151-** | **I 151-E 391** | **I 151-D 402** | **I 151-S 412** | **I 151-S 422** |
| #**A** | | | | | | | * | * |
| #**B** | | | | | | | * | * |
| #**C** | | | | | | | * | * |
| #**D** | | | | | * | * | * | * |
| #**E** | | | | | | | * | * |
| #**F** | * | * | * | * | * | * | * | * |
| #**G** | | | | | | | * | * |
| #**K** | | | | | * | * | * | * |
| #**M** | | | | | | | * | * |
| #**N** | | | | | * | * | * | * |
| #**O** | | | | | | | * | * |
| #**Q** | | | | | | | * | * |
| #**S** | | | | | | | * | * |
| #**T** | | | | | | | * | * |
| #**U** | | | | | | | * | * |
| #**V** | | | | | * | * | * | * |
| #**X** | | | | | * | * | * | * |
| #**Y** | | | | | | | * | * |

Then the selection method (IVEM) allows a rapid identification of epitopes recognized *in vivo* by ScFvs previously selected with the IACT method, thus representing a valid alternative to traditional expensive and time consuming methods. Moreover only by means of the instant technique an easy and precise identification of the *in vivo* recognizing antigen region is achieved.

### BIBLIOGRAPHY

- Barbacid (1987) ras genes. Annu. Rev. Biochem. **56**, 779-828.
- Biocca, S. & Cattaneo, A. (1995). Intracellular immunization: antibody targeting to subcellular compartments. *Trends Cell Biol* 5, 248-252.
- Brent, R. & Pthashne, M. (1985) A eukaryotic transcriptional activator bearing the DNA specificity of a prokaryotic repressor. *Cell* **43** (3 Pt 2), 729-36.
- Carlson, J. R. (1988). A new means of inducibly inactivating a cellular protein. *Mol Cell Biol* **8**(6), 2638-46.
- Cattaneo, A. & Biocca, S. (1997). Intracellular Anribodies: Development and Applications (Springer, Ed.).
- Cattaneo, A. & Biocca, S. (1999). The selection of intracellular antibodies. *Trends Biotechnol* **17**(3), 115-21.
- Cattaneo, A & Neuberger, M.S. (1987). Polymeric immunoglobulin M is secreted by transfectants of non-lymphoid cells in the absence of immunoglobulin J chain. *Embo J* **6**(9), 2753-8.
- Fasulo, A. et al. (2000). J. Neurochem. **75**, 624-633.
- Gietz, D., et al., (1992). Improved method for high efficiency transformation of intact yeast cells. *Nucleic Acids Res* **20**(6), 1425.
- Goedert et al., (1999). Tau gene mutation in familial progressive subcortical gliosis. *Nature Medicine,* **5**, 454-457.
- Golemis, E.A. & Brent, R. (1992). Fused protein domains inhibit DNA binding by LexA. *Mol Cell Biol* **12**(7), 3006-14
- Grand and Owen (1991). The biochemistry of ras p21. *Biochem. J.* 279, 609-631.
- Hill, J. et al., (1991). DMSO-enhanced whole cell yeast transformation [published erratum appears in Nuclei Acids Res 1991 Dec 11; 19(23):6688] *Nucleic Acids Res* **19**(20), 5791.
- Hollenberg, S.M., et al., (1995). Identification of a new family of tissue-specific basic helix-loop-helix proteins with a two-hybrid system. *Molecular and Cellular Biology* **15**(7), 3813-3822.
- Hong, M., et al. (1998). Mutation-specific functional impairments in Distinct Tau isoforms of Heriditary FTDP-17. *Science,* 282, 1914-1917.
- Marasco, W.A. (1997). Intrabodies: turning the humoral immune system outside in for intracellular immunization. *Gene Ther* 4(1), 11-5.
- Novak, M. et al. (1991). Difference between the tau protein of Alzheimer paired helical filament core and normal tau revealed by epitope analysis of monoclonal antibodies 423 and 7.51. *Proc Natl Acad Sci USA* **88**(13), 5837-41.
- Sambrook, J., Fritsch, E. F. & Maniatis, T (1990). *Molecular Cloning: A Laboratory Manual.* Cold Spring Harbor NY edit (Press, C.S.H.I., Ed.)
- Schiestl, R.H. & Gietz, R.D. (1989). High efficiency transformation of intact yeast cells using single stranded nucleic acids as a carrier *Curr Gen* 16(5-6), 339-46.
- Silver, P.A., Brent, R. & Ptashne, M. (1986). DNA binding is not sufficient for nuclear localization of regulatory proteins in Saccharomyces cerevisiae. *Mol Cell Biol* 6(12), 4763-6.
- Spillantini, M.G. et al. (1998). Mutation in the gene in familial multiple system taupathy with presenile dementia. *Proc. Natl. Acad. Sci USA,* **95,** 7737-7741.
- Visintin et al. (1999). Selection of antibodies for intracellular function using a two-hybrid system. *Proc. Natl. Acad. Sci. USA* **96,** 11723-11728.

## Claims

1. Method for identification of epitopes of the tau protein able to bind intracellularly to a specific antibody comprising the steps of :
a) co-transforming cells by means of a first vector including the nucleotide sequence encoding for the region of an anti-tau antibody able to recognise and bind intracellularly the tau protein and by a second vector family, each member of the family comprising a nucleotide sequence encoding for a peptide and forming a peptide library as a whole;
b) growing co-transformed cells in such an environment that only cells wherein the antibody region and the peptide recognise and interact each other are able to replicate and/or be recognised because: the antibody region able to recognise and bind intracellularly the peptide is associated with a first molecule; the peptide is associated with a second molecule; the interaction between the first and the second molecule generates a selectable phenotype and/or a recognisable signal; and the interaction between the first and the second molecule occurs only when the antibody region and the peptide recognise and interact each other;
c) selecting cells and identifying the peptide as epitope.

2. Method for identification of epitopes of the tau protein able to bind intracellularly to a specific antibody according to claim 1 wherein the peptide library is a library of tau peptide fragments; or phylogenetic variants or pathology associated mutants or splicing isoforms thereof.

3. Method for identification of epitopes of the tau protein able to bind intracellularly to a specific antibody according to claim 1 or 2 wherein the cells are yeast cells.

## Patentansprüche

1. Verfahren zum Identifizieren von Epitopen des tau-Proteins, die intrazellulär an einen spezifischen Antikörper binden können, umfassend:
a) Cotransformieren von Zellen mittels eines ersten Vektors, der die Nukleotidsequenz enthält, die die Region eines Anti-tau-Antikörpers kodiert, die das tau-Protein erkennen und intrazellulär daran binden kann, und mittels einer zweiten Vektorfamilie, wobei jedes Mitglied der Familie eine Nukleotidsequenz umfasst, die ein Peptid kodiert, wobei eine Peptidbibliothek als Ganzes gebildet wird;
b) Züchten cotransformierter Zellen in einer derartigen Umgebung, dass nur Zellen, bei denen die Antikörperregion und das Peptid einander erkennen und miteinander in Wechselwirkung treten, sich replizieren können und/oder erkennen können, weil die Antikörperregion, die das Peptid erkennen und intrazellulär daran binden kann, mit einem ersten Molekül assoziiert ist; das Peptid mit einem zweiten Molekül assoziiert ist; die Wechselwirkung zwischen dem ersten und dem zweiten Molekül einen selektierbaren Phänotyp und/oder ein erkennbares Signal erzeugt; und die Wechselwirkung zwischen dem ersten und dem zweiten Molekül nur erfolgt, wenn die Antikörperregion und das Peptid einander erkennen und miteinander in Wechselwirkung treten;
c) Selektieren der Zellen und Identifizieren des Peptids als Epitop.

2. Verfahren zum Identifizieren von Epitopen des tau-Proteins, die intrazellulär an einen spezifischen Antikörper binden können, nach Anspruch 1, wobei die Peptidbibliothek eine Bibliothek von tau-Peptidfragmenten oder phylogenetischen Varianten oder mit Pathologie zusammenhängenden Mutanten oder Splicing-Isoformen davon ist.

3. Verfahren zum Identifizieren von Epitopen des tau-Proteins, die intrazellulär an einen spezifischen Antikörper binden können, nach Anspruch 1 oder 2, wobei die Zellen Hefezellen sind.

## Revendications

1. Procédé d'identification des épitopes de la protéine tau capables de se lier de manière intracellulaire à un anticorps spécifique comprenant les étapes consistant à :
a) co-transformer les cellules au moyen d'un premier vecteur comprenant la séquence nucléotide codante pour la région d'un anticorps anti-tau capable de reconnaître et de lier de manière intracellulaire la protéine tau et par une deuxième famille de vecteurs, chaque membre de la famille comprenant une séquence nucléotide codante pour un peptide et formant globalement une banque de peptides ;
b) mettre en culture les cellules co-transformées dans un environnement tel que seules les cellules dans lesquelles la région d'anticorps et le peptide se reconnaissent et interagissent l'un envers l'autre sont capables de répliquer et/ou d'être reconnues parce que : la région d'anticorps capable de reconnaître et de lier de manière intracellulaire le peptide est associée à une première molécule ; le peptide est associé à une deuxième molécule ; l'interaction entre la première et la deuxième molécule génère un phénotype de sélection spécifique et/ou un signal reconnaissable ; et l'interaction entre la première et la deuxième molécule se produit uniquement quand la région d'anticorps et le peptide se reconnaissent et interagissent l'un envers l'autre ;
c) sélectionner des cellules et identifier le peptide en tant qu'épitope.

2. Procédé d'identification des épitopes de la protéine tau capables de se lier de manière intracellulaire à un anticorps spécifique selon la revendication 1, dans lequel la banque de peptides est une banque de fragments de peptides tau ; ou des variantes phylogénétiques ou des mutants associés à une pathologie ou des isoformes d'épissages de celle-ci.

3. Procédé d'identification des épitopes de la protéine tau capables de se lier de manière intracellulaire à un anticorps spécifique selon la revendication 1 ou la revendication 2, dans lequel les cellules sont des cellules de levure.
